(19) Europäisches Patentamt
European Patent Office
Office européen des brevets

(11) Publication number: 0 352 007 A2

(12) EUROPEAN PATENT APPLICATION

(21) Application number: 89307043.3

(51) Int. Cl.4: C07C 255/09 , C07C 253/30

(22) Date of filing: 11.07.89

(30) Priority: 18.07.88 GB 8817030

(43) Date of publication of application:
24.01.90 Bulletin 90/04

(84) Designated Contracting States:
BE DE ES FR GB IT NL

(71) Applicant: IMPERIAL CHEMICAL INDUSTRIES PLC
Imperial Chemical House Millbank
London SW1P 3JF(GB)

(72) Inventor: Shaw, Gordon
103 Farndale Drive Pine Hills
Guisborough Cleveland(GB)

(74) Representative: Locke, Timothy John et al
Imperial Chemical Industries PLC Legal Department: Patents Bessemer Road PO Box 6
Welwyn Garden City Hertfordshire, AL7 1HD(GB)

(54) **Dimerisation process.**

(57) Acrylonitrile is dimerised in the presence of 10 to 60% by weight of a phosphorus III compound which has at least one hydrocarbyl group and at least one alkoxy or cycloalkoxy group and a proton-donating solvent.

EP 0 352 007 A2

## Dimerisation Process

This invention relates to a dimerisation process and, especially, to a process for the dimerisation of acrylonitrile to linear $C_6$ dinitriles.

In our US Patent No 4,126,632 we have described a process for the dimerisation of acrylonitrile to predominantly straight-chain $C_6$ dimers comprising contacting the acrylonitrile with an organic phosphorus (III) compound which has at least one hydrocarbyl and at least one alkoxy or cycloalkoxy group attached to the phosphorus atom or atoms, the acrylonitrile being dissolved in an organic solvent capable of donating protons and the acrylonitrile and solvent being substantially dry.

A concentration of the catalyst of 0.01 to 5% by volume was disclosed. We have now found that considerably higher concentrations may be used with considerable benefit to the rate of reaction and with surprisingly little average effect on selectivity to the desired products.

According to the present invention, a process for the dimerisation of acrylonitrile to predominantly straight chain $C_6$ dimers comprises contacting the acrylonitrile with at least one organic phosphorus (III) compound which has at least one substituted or unsubstituted hydrocarbyl, preferably aryl group and at least one alkoxy or cycloalkoxy group attached to the phosphorus atom or atoms, the acrylonitrile being dissolved in an organic solvent capable of donating protons and the acrylonitrile and solvent being substantially dry the organic phosphorus (III) compound(s) comprising 10 or preferably 20 to 60% and preferably 20 to 35% of the total material present by weight during the dimerisation.

Suitable organic phosphorus (III) compounds include those of general formulae:-

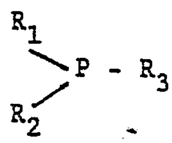 or 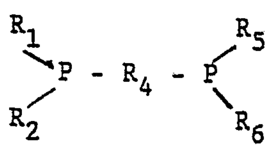

where $R_1$ and $R_5$ are individually hydrocarbyl groups, $R_2$ and $R_6$ are individually alkoxy or cycloalkoxy groups, $R_3$ is hydrocarbyl, alkoxy or cycloalkoxy group or other monovalent radical, and $R_4$ is a divalent hydrocarbyl, hydrocarbyloxy or other di-functional group. It is also possible that one or more groups $R_1$ to $R_3$ may form part of one or more ring systems.

The hydrocarbyl groups may be aryl, alkyl, alkaryl, aralkyl or cycloalkyl.

The phosphorus (III) compound is preferably of formula

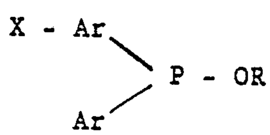 (i) 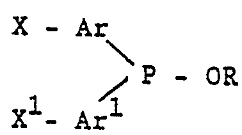 or

(iii) 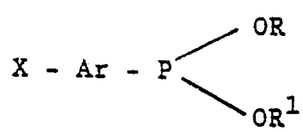

where Ar and $Ar^1$ are individually aromatic nuclei, eg phenyl or naphthyl, and where groups X and $X^1$, which may be the same or different, are electron donating substituents of the aromatic nucleus which give rise to a negative Hammet $\sigma$ - constant; and R and $R^1$ represent alkyl or cycloalkyl groups. Substituents X and $X^1$ may be in the para or meta positions; but are preferably para. In the case of compounds of formula (ii) above, substituents X and $X^1$ may be para in one Ar or $Ar^1$ group but meta in the other.

It will be appreciated that the phosphorus (III) compounds defined above are either phosphinites or phosphonites.

A discussion on Hammett $\sigma$ - constants and a table showing values for most common substituents is to be found in an article by Clark and Perrin in Quarterly Reviews, Vol 18, 1964 pp 295-320.

Examples of suitable substituents X include alkoxy groups, eg methoxy, ethoxy, i-propoxy and t-butoxy; alkyl groups, eg methyl, ethyl and propyl; and alkyl amino groups, eg dimethylamino and diethylamino. The alkoxy, alkyl and alkylamino groups preferably contain from 1 to 8 carbon atoms. It is essential that group X should be one which does not react adversely with the components of the reaction system.

Suitable groups R and $R^1$ include alkyl groups having 1 to 8 carbon atoms for example methyl, ethyl, isopropyl, neopentyl, 2-ethylhexyl; and cycloalkyl groups for example cyclohexyl.

High proportions of straight-chain dimers are obtained where each hydrocarbyl group is aryl, in the case where either 1 or 2 hydrocarbyl groups are present in the phosphorus (III) compound. High reaction rates are obtained where one or both of the hydrocarbyl groups is alkyl in cases where two hydrocarbyl groups are present; but these high reaction rates may be accompanied by somewhat lower proportions of straight-chain dimers than where both hydrocarbyl group are simple aryl. The hydrocarbyl groups may contain substituent, suitable substituent groups being halogen, cyanide, alkyl and alkoxy. High rates may also be achieved by the use of substituted aryl groups, for example, p-methoxyphenyl. The alkoxy or cycloalkoxy group or groups may contain similar substituents and may also contain aryl substituents. Examples of suitable alkoxy groups include methoxy, ethoxy, benzyloxy and isopropoxy.

Suitable examples of divalent groups $R_4$ include alkylene, polyalkylene and phenylene and poly phenylene groups, alkylene dioxy and polyalkylene dioxy groups.

Groups $R_1$ to $R_4$ may also be part of a polymeric backbone, for example polystyrene or polyvinyl alcohol, or be linked to an inorganic support, for example silica or alumina.

Provided that the groups $R_1$ to $R_4$ do not give rise to undesirable steric effects there is no finite limit on the number of carbon atoms that they may contain. However they will commonly contain from 1 to 10 carbon atoms.

It will be appreciated from the above that in the phosphorus (III) compounds which are useful in our invention each phosphorus atom must have at least one substituted or unsubstituted hydrocarbyl group and one alkoxy group attached to it, but must not bear either only said hydrocarbyl or only alkoxy groups.

Examples of suitable phosphorus (III) compounds include diethyl phenylphosphonite, dimethyl phenylphosphonite, dimethyl p- methylphenylphosphonite, diethyl p-methylphenylphosphonite, methyl diphenylphosphinite, isopropyl diphenylphosphinite, ethyl phenylethylphosphinite, ethyl diphenylphosphinite, cyclohexyl diphenylphosphinite, diethyl p-tolylphosphinite, 2-ethylhexyl diphenylphosphinite, bis(2-ethylhexyl) phenylphosphonite, di(isopropyl) phenylphosphonite, di(neopentyl) phenylphosphonite, 2-octyl diphenylphosphonite, bis(3,5,5-trimethylhexyl) phenylphosphonite, 2-methylcyclohexyl diphenylphosphinite, 3,5,5-trimethylhexyl diphenylphosphinite, sec butyl diphenylphosphinite, cyclohexyl diphenylphosphinite, benzyl diphenylphosphinite, and $Ph_2PO(CH_2)_4OPPh_2$

$Ph_2POCH\ (-CH_2)_2\ CHOPPh_2$
Me Me

and

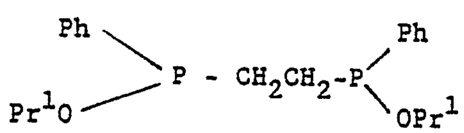

The presence of an organic solvent is essential to our process, since in the absence of solvent rapid polymerisation of the acrylonitrile occurs. Suitable solvents are proton donating solvents which are substantially unreactive in respect of addition to, or reaction with, the unsaturated linkage of the acrylonitrile or the products of acrylonitrile dimerisation. Furthermore, the solvent must not react with the phosphorus compounds or catalytic intermediates to form inactive phosphorus species at such a rate as to seriously impair the dimerisation reaction. For example, phenols have been found to be unsuitable in this respect.

Preferably hydroxylic solvents, for example alcohols preferably having 1 to 10 carbon atoms, are used, provided always that they do not react adversely with the phosphorus compound or any intermediates it may form with acrylonitrile. This may be readily established by experiment. Secondary alcohols are preferred, for example, 2-butanol and isopropanol.

The concentration of proton-donating solvent is generally in the range 1 to 20% and preferably 5 to 15% by volume, calculated on the total volume of the reaction medium, but the optimum concentration will vary with the precise nature of the solvent and the catalyst compound. The molar concentration of proton donating solvent will generally be in a ratio of 3:1 to 0.5:1 to the number of gram atoms of the phosphorus (III) present as the phosphorus (III) compound.

In order to reduce the amount of hexamer and/or other oligomers or polymers (hereafter referred collectively as polymeric by-products or merely polymers) which may be co-produced with the desired dimeric products, it is often desirable to add an inert, non-hydroxylic co-solvent to the reaction mixture used in our process. It will be apparent that the co-solvent must be dried to a level which maintains the overall

anhydrous state of the system.

Suitable non-hydroxylic organic solvents include hydrocarbons, for example, hexane, cyclohexane, toluene, and petroleum ethers; ethers, for example, tetrahydrofuran, diethyl ether and diisopropyl ether; and nitriles, for example, acetonitrile, propionitrile; and fluorobenzenes. The hydrocarbon co-solvents are generally preferred.

An essential feature of the present invention is that the reaction must be conducted in the substantial absence of water. Without prejudice to our invention, we believe that the water reacts with the catalyst in the presence of acrylonitrile to give a non-catalytic addition compounds. Thus, the acrylonitrile, proton-donating solvent and co-solvent must be dried before use, otherwise the reaction may be completely inhibited. In particular acrylonitrile, which commonly contains as much as 4000 ppm of water, even after distillation, must be rigorously dried. It is also noted that hydroquinone stabilisers, which are normally present in acrylonitrile as supplied, need not be removed. For example, if the reactants contain 300 ppm of water, reaction is seriously inhibited at a concentration of the phosphorus compound of 0.5% by volume; but at water concentrations of 50 ppm or lower reaction takes place readily. Any suitable drying technique may be used, provided that the final water level is sufficiently low. For example, acrylonitrile and hydroxylic solvents may be dried by being contacted with calcium hydride or a 3A or 4A molecular sieve. It will be appreciated that at higher concentration of water, eg 300 ppm, it will be possible to cause the reaction to proceed by adding much larger amounts of catalyst. If this is done, selectivity is generally unaffected; but the process would become less attractive because of high catalyst usage.

The concentration of acrylonitrile in the solvent or solvent mixture generally should range from 1 to 35% by volume. The concentration of acrylonitrile is kept as high as possible in order to optimise throughput and thus concentration in the range 10 to 30% by volume are generally preferred.

It is preferred that the co-solvent should comprise benzene, an alkyl benzene for example toluene or ethyl benzene, and/or a xylene.

Suitably the reaction medium comprises, per part (by weight) of acrylonitrile, 1 to 10 parts of co-solvent, 0.2 to 1 part of organic proton-donating solvent, and 1 to 5 parts of catalyst (by weight). Suitably at least 1 part of the proton donating solvent is present per 10 parts of catalyst, and preferably 3 to 10 moles of acrylonitrile are present per mole of catalyst.

Changes in the ratio of proton-donating solvent/co-solvent are generally reflected by changes in the amount of polymers formed and changes in the reaction rate.

The reaction temperature is commonly in the range 0° to 120°C; but it is generally preferred to keep the range temperature below 75°C, to minimise polymerisation of the acrylonitrile and dimeric products. Preferably, the reaction temperature is in the range 20° to 70°C. It is noted that the reaction will proceed below 0°C, maintaining selectivity, but at a reduced rate.

Unlike other acrylonitrile dimerisation processes, the presence of compounds such as hydroquinone and its monomethyl ether, p-methoxyphenol, which are commonly used at present as acrylonitrile stabilisers, should be avoided.

The reaction may be carried out batchwise or continuously. In the latter case it may be convenient to support the catalyst compound or to use a polymeric tervalent phosphorus compound to enable the reaction to be carried out in the liquid phase using a heterogeneous catalyst.

The dimeric products of our invention are predominantly linear $C_6$ dinitriles, especially the 1,4-dicyanobutenes.

The desired products may be readily separated from the reaction mixture for example by fractional distillation or solvent extraction.

Example 1 (Comparative)

Isopropyl bis-p-tolylphosphinite (0.5 parts by volume) was added at room temperature to a mixture of benzene (10 parts) acrylonitrile (2 parts) and isopropanol (1 part), which had been made up in a glass reactor with the rigorous exclusion of air and moisture. The reactor vessel was then immersed in a thermostatted oil-bath for four hours at 40°C. 0.11 ml of 60% hydrogen peroxide was then added to decompose the phosphinite and thus terminate the reaction and the reaction mixture analysed by gas chromatography. The acrylonitrile conversion amounted to 78.9%. The yield to linear dimers (DCB) was 89.6% while the yield to the branched dimer (MEGN) was 4.5%. The remaining yield loss of 5.9% was made up of acrylonitrile oligomers and acrylonitrile hexamer.

## Examples 2 - 11

The procedure of Example 1 was repeated using identical quantities of acrylonitrile and isopropanol but with increasing levels of catalyst and maintaining constant reaction volume by reducing the amount of benzene. The reaction times at 40°C were chosen to give an acrylonitrile conversion around 80%. The results from these Examples along with those from Example 1 are shown in the Table.

TABLE

| EXAMPLE NUMBER | 1 | 2 | 3 | 4 | 5 | 6 | 7 | 8 | 9 | 10 | 11 |
|---|---|---|---|---|---|---|---|---|---|---|---|
| INITIAL COMPOSITION % w/w | | | | | | | | | | | |
| Benzene | 75.14 | 70.82 | 66.89 | 62.26 | 58.83 | 55.02 | 50.67 | 45.62 | 39.48 | 31.92 | 24.32 |
| Acrylonitrile | 14.04 | 13.88 | 13.89 | 13.66 | 13.64 | 13.66 | 13.47 | 13.53 | 13.31 | 13.19 | 12.94 |
| Isopropanol | 6.75 | 6.69 | 6.67 | 6.62 | 6.60 | 6.59 | 6.50 | 6.55 | 6.45 | 6.40 | 6.24 |
| Catalyst | 4.07 | 8.60 | 12.54 | 17.06 | 20.92 | 24.72 | 29.35 | 34.30 | 40.76 | 48.50 | 56.5 |
| Reaction Time | 4 hrs | 2 hrs | 1 hr 20 mins | 1 hr | 48mins | 40mins | 34mins | 29mins | 24mins | 20.5 mins | 17mins |
| % PRODUCT YIELD w/w | | | | | | | | | | | |
| DCB[a] | 89.6 | 88.8 | 89.4 | 88.8 | 89.0 | 88.7 | 87.2 | 87.4 | 83.0 | 86.4 | 78.6 |
| MEGN[b] | 4.5 | 4.6 | 4.6 | 4.5 | 4.4 | 4.5 | 4.4 | 4.7 | 4.2 | 4.6 | 4.3 |
| AN* / CATALYST | 17.7 | 8.28 | 5.68 | 4.11 | 3.35 | 2.83 | 2.35 | 2.02 | 1.67 | 1.39 | 1.17 |
| * molar ratio | | | | | | | | | | | |
| % AN CONVERSION | 78.9 | 75.9 | 80.7 | 80.3 | 79.3 | 79.0 | 82.7 | 84.1 | 84.5 | 90.8 | 87.5 |

a) Total linear dimers comprising cis-dicyanobutene-1, trans-dicyanobutene-1 and trans-dicyanobutene-2

b) Branched dimer, 2-methylene glutaronitrile.

AN means acrylonitrile.

## Claims

1. A process for the dimerisation of acrylonitrile to predominantly straight chain $C_6$ dimers which comprises contacting the acrylonitrile with at least one organic phosphorus (III) compound which has at least one substituted or unsubstituted hydrocarbyl group, and at least one alkoxy or cycloalkoxy group attached to the phosphorus atom or atoms, the acrylonitrile being dissolved in an organic solvent capable of donating protons and the acrylonitrile and solvent being substantially dry, the organic phosphorous (III) compound(s) comprising 10 to 60% of the total material present by weight during the dimerisation.

2. A process according to claim 1 in which the organic phosphorus (III) compound(s) comprises 20 to 35% of the total material present by weight during the dimerisation.

3. A process according to claim 1 or 2 in which the organic phosphorus (III) compound(s) has the general formula:-

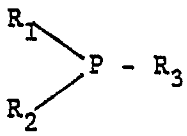 or 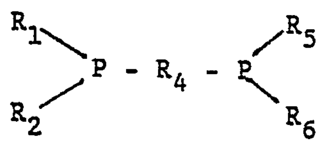

where $R_1$ and $R_5$ are individually hydrocarbyl groups, $R_2$ and $R_6$ are individually alkoxy or cycloalkoxy groups, $R_3$ is hydrocarbyl, alkoxy or cycloalkoxy group or other monovalent radical, and $R_4$ is a divalent hydrocarbyl, hydrocarbyloxy or other di-functional group.

4. A process as claimed in any preceding claim in which the phosphorus (III) compound is of formula

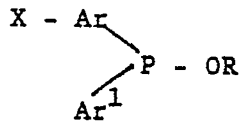 (i) 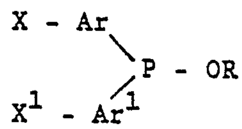 or

(iii) 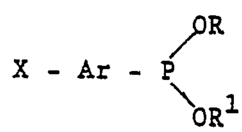

where Ar and $Ar^1$ are individually aromatic nuclei, and where groups X and $X^1$, which may be the same or different, are electron donating substituents of the aromatic nucleus which give rise to a negative Hammet $\sigma$ - constant; and R and $R^1$ represent alkyl or cycloalkyl groups.

5. A process as claimed in claim 4 in which the groups X and $X^1$ are alkoxy, alkyl and/or alkylamino groups having 1 to 8 carbon atoms in the para position(s).

6. A process as claimed in any preceding claim in which the solvent comprises an alcohol having 1 to 10 carbon atoms.

7. A process as claimed in claim 6 in which the alcohol is a secondary or tertiary alcohol.

8. A process as claimed in any preceding claim in which 5 to 15% by volume of proton donating solvent is present based on the total volume.

9. A process according to any preceding claim in which an inert non hydroxylic co-solvent is present.

10. A process according to any preceding claim in which 10 to 30% by volume of acrylonitrile is present.

11. A process according to any preceding claim in which the reaction medium comprises per part by weight of acrylonitrile, 1 to 10 parts of co-solvent, 0.2 to 1 part of organic proton donating solvent and 1 to 5 parts of catalyst.

12. A process as claimed in any preceding claim which is carried out at 20 to 70 °C.